# EUROPEAN PATENT SPECIFICATION

(11) **EP 4 611 640 B1**
(45) Date of publication and mention of the grant of the patent: **29.10.2025**
(21) Application number: 24758773.6
(22) Date of filing: 20.08.2024
(51) Int. Cl.: A61B 6/04, A61B 5/00, A61B 5/055, A61B 6/03, A61B 90/00

(54) **MEDICAL IMAGING SYSTEM**
MEDIZINISCHES BILDGEBUNGSSYSTEM
SYSTÈME D'IMAGERIE MÉDICALE

(30) Priority: 29.08.2023 EP 23193823
(43) Date of publication of application: 10.09.2025
(73) Proprietor: Koninklijke Philips N.V., 5656 AG Eindhoven (NL)
(72) Inventor: SMINK, Jouke, 5656 AG Eindhoven (NL); KOKEN, Peter, 5656 AG Eindhoven (NL); STEHNING, Christian, 5656 AG Eindhoven (NL); BECK, Gabriele, 5656 AG Eindhoven (NL)
(74) Representative: Philips Intellectual Property & Standards
(86) International application number: PCT/EP2024/073309
(87) International publication number: WO 2025/045652

(56) References cited:
- US-A- 6 041 249
- US-A1- 2017 281 294
- US-A1- 2020 057 123

## Description

### FIELD OF THE INVENTION

The invention relates to an imaging system for medical imaging, in particular to an imaging system for an image-guided workflow. The invention further relates to a computer-implemented method for controlling a table with respect to an imaging system. The invention also relates to a computer program element, and to a computer readable medium having stored thereon the computer program element.

### BACKGROUND OF THE INVENTION

US2017/281294A1 describes a marking method and a computerized tomography apparatus, the method comprising: scanning a target object to obtain an image of the target object; determining an intervention position on a surface of the target object according to a position of a part of interest in the image of the target object; marking the intervention position on the surface of the target object.

US6041249A discloses a computed tomography apparatus equipped with a device for marking a guide path on a patient for a medical instrument to be used in a medical procedure, such as a puncturing needle. The computed tomography apparatus produces a planning image, and a guide path is identified within the planning image. A computer, using the planning image and the path identified thereon, automatically adjusts a position of a light source, and if necessary a patient table on which a patient is supported, so that a beam from the light source is positioned to coincide with the guide path identified on the image.

US2020/057123A1 discloses a system for guiding a medical intervention. More particularly a system for guiding invasive devices such as biopsy needles using Magnetic Resonance, Computed Tomography, or other types of images. The system employs a device guide that operates on the surface of a sphere that is centered on a selected target.

Large imaging systems such as Magnetic Resonance Imaging (MRI) or X-ray Computed Tomography (CT) may provide superb clinical imaging for diagnosis and therapy. However, access to the patient is a potential drawback of image-guided interventions utilizing such imaging systems because the area of interest is typically placed in the center of a long and narrow bore during imaging. This may be of particular importance for needle interventions, such as for biopsies or localized treatment, where it may be a challenge to reach inside the imaging system bore. As an example, a surgeon may have to bow into the bore to with (real-time) imaging check a location for incision. This may be done by pushing the finger into the potential incision region. After needle placement this location may not be accurate enough and a replacement of the needle may be required.

Image-guided procedures on patients being imaged with an imaging system having a bore, such as MRI or CT, may therefore be inefficient and result in unsatisfactory interventionalist ergonomics with uncomfortable and/or impractical working positions. Furthermore, other aspects such as patient throughput, and ultimately medical outcomes may also be sub-optimal.

Hence, there is a need to improve the workflows of such image guided interventions.

### SUMMARY OF THE INVENTION

It is an object of the invention to provide an improved image guided workflow. The invention is defined by the independent claims. Advantageous embodiments are defined in the dependent claims.

According to a first aspect, there is provided an imaging system for medical imaging. The imaging system comprises: a bore for receiving an object to be imaged; a table for supporting the object and configured to move longitudinally with respect to the bore; a position indicator for indicating a position; and a controller. The controller may e.g. comprise a memory and a processor. The controller is configured to:
- receive an image, acquired by the imaging system, of a region of interest of the object on the table, wherein the table is positioned in a stored first table position, wherein in the first table position the region of interest of the object is at a reference position inside the bore;
- receive user input marking an intervention location in the image;
- determine a puncture location on the object relative to the reference position; and
- calculate and store a second table position, based on the puncture location, the position indicated by the position indicator, and the stored first table position, such that when the table is in the second table position, the puncture location on the object coincides with the position indicated with the position indicator.

By determining a puncture (or incision or similar) location on the object (such as a patient) based on user input to the image and subsequently calculating a second table position where that location is marked with the position indicator, the efficiency of a workflow may be improved. The second table position can be automatically determined. When the table is moved to the second table position, the puncture location is indicated with the position indicator. Preferably the table may be automatically moved by the controller, but other options are also possible. Such as manual or semi-automatic movement of the table to a determined coordinate. The second position is preferably such that the puncture location of the object on the table in the second position is outside of the bore of the imaging system. In this way, a user does not have to stretch into the bore to see the indication of the puncture location.

The controller is further configured to store a third table position, wherein when the table is in the third table position the table is further outside of the bore compared to in the first position, and wherein the region of interest of the object on the table in the third table position is not at an isocenter of the bore. The third table position is different from the first table position and from the second table position. The third table position may be received or calculated or adapted by the controller, e.g. from selection or other input by a user, and/or may be predetermined. The third table position may be selected or calculated based on ergonomic parameters, such as a length of the user, a reach of the user, a height of the table, a length of the object, a length of an instrument etc. It is advantageous to store a third table position where e.g. an interventionalist has ergonomically better access to the object on the table compared to the first table position, since the table is further outside the bore. Furthermore, since the third table position is different from the second table position, movement freedom of the interventionalist is not limited by the position indicator or related structures, such as a laser bow. The third table position may be further out from the the iso-center of the bore compared to the second table position, e.g. to improve movement freedom. Alternatively, the third table position maybe closer to the iso-center of the bore than the second table position. The third table position may be close to the first table position, further out of the bore compared to the first table position in order to provide improved ergonomics for the interventionalist when accessing e.g. the puncture location, but close enough such that table movements advantageously can be limited and/or off-center imaging in the third position may be enabled.

Since the first position and the third position are stored, the interventionalist may during an image guided procedure advantageously toggle the table position between the third position for good access to the object (patient) and the first position for optimal imaging of the object (patient) to check the progress. Preferably, the table is automatically moved between said stored table positions, such as at a command by the interventionalist via a user interface. Herewith, both the efficency of the workflow and the ergonomics of the interventionalist may be improved.

The reference position inside the bore may be at an isocenter of the bore or close to an isocenter of the bore for optimal image acquisition of the region of interest.

Determining a puncture location relative to the reference position may comprise receiving user input to mark the puncture location. Such as e.g. receiving user input to the image with the marked intervention location. As an example, the user may indicate a suitable path between the intervention location and a puncture location. Alternatively, or additionally, the controller may translate the marked intervention location in the image to a puncture location on the object relative to the reference position via computational road-mapping of the internal volume of the object on the table. The translation may comprise to calculate, based on an imaged volume of the object, an optimal path for an intervention needle, catheter or similar instrument from the puncture location on the object to the intervention location inside the object. Translating the intervention location in the image to determine a puncture location on the object may be based on anatomical regions that can be passed or needs to be avoided by e.g. a catheter. Determination of a puncture location may include the use of artificial intelligence algorithms, such that algorithms that can learn from previous intervention procedures and/or strategies. The controller may be configured to automatically determine the puncture location or provide a recommendation for a user providing input.

Hence, machine learning or artificial intelligence may be integrated for providing and suggesting an optimal puncture location and trajectory to the surgeon. Data mining taking a large set of image guided interventions into account can be used to determine and integrate puncture locations and catheter trajectories used in clinical practice into an artificial intelligence network, such as e.g. a deep learning network. Personalized preferences including avoidance of anatomical and physiological areas may be taken into account. Similarly, machine learning or artificial intelligence may be employed to determine a suitable third table position, e.g. based on user, object and/or system parameters.

The position indicator may be a stationary indicator, such as but not limited to a laser (light) bow or bridge, e.g. positioned close to the bore of the imaging system. Other stationary solutions, such as position indicators attached to the bore of the imaging system or to a wall, a floor, or a roof of the imaging room etc. are also possible. The position indicator in the form of a laser bridge (light visor) may be a standard laser bridge (light visor), commonly used for patient positioning e.g. for therapy or therapy planning, or a similar existing laser or other light source attached on or in proximity to the imaging system. This is advantageous, since it may enable to indicate the puncture location on the object in the second table position without additional hardware.

Alternatively, the position indicator may be mobile. Such as but not limited to a position indicator attached to a mobile unit like a robotic device. E.g. a robotic device for performing (part of) the intervention. An intervention robotic device may be positioned on a rail mounted on the table, which allows movement of the intervention robotic device to the incision and treatment region. An improved positioning of the intervention robotic device may be accomplished with the position indicated by the position indicator overlapping with a translated incision location of the robotic device. The position indicator may use a laser or other light sources to indicate e.g. a spot or a line on the object on the table.

The controller may e.g. comprise a processor and a memory. The processor may be a single and/or multi core processing unit, a graphics processing unit, an accelerated processing unit, a digital signal processor, a field programmable gate array, and/or an application-specific integrated circuit, etc. Processing may be carried out involving a single apparatus, such as e.g. a single controller comprising a processor, or may be carried out by a distributed system with multiple local and/or remote units. The memory may be a short-term and/or a long-term memory configured to interact with the processor.

According to an embodiment of the invention, the controller is configured to calculate and store an updated coordinate for the position indicator, such that when the table is in the second table position and the position indicator indicates an updated position with the updated coordinate, the puncture location on the object coincides with the updated position indicated with the position indicator. This is advantageous in cases the position that is indicated with the position indicator can be updated, such as by moving a mobile position indicator and/or changing the target spot or line that the indicator marks. Such as by pivoting an indicator mounted on e.g. a wall or roof. When the position indicated by the position indicator can move, it may be possible to reduce the absolute movement of the patient table, speed up the workflow and provide additional flexibility. As an example, a mobile position indicator such as a robotic device, may move into the bore of the imaging system. E.g. the system may have rails on which the position indicator can move with respect to the bore and/or the table. When the position indicator on a robotic surgical device may move into the bore, the second table position may advantageously be similar or the same to the first table position such that table movement is reduced. Also in the case when a robotic surgical device is used to perform (part of) an intervention at the second table position, the stored third table position in combination with the stored first table position provides a user such as an interventionalist with an ergonomic position to e.g. perform further surgical steps, fine-tune the intervention, toggle the table back and forth to verify the progress of the intervention hands on and toggled with imaging etc.

According to an embodiment of the invention, the controller is configured to store a temporary table position, wherein when the table is in the temporary table position the region of interest coincides with the position indicated with the position indicator, and wherein the controller is configured to calculate and store the first table position based on the temporary table position and an offset between the position indicated with the position indicator and the reference position. **In** this way, the system may automatically determine the first table position. An operator may move the table to the temporary table position such that the region of interest that the operator wants to image is indicated with the position indicator. This may be very intuitive for the operator since the region is directly indicated e.g. under a laser bridge. Once the table is in the correct temporary position, that position may be stored, and the first table position may be automatically determined. The first table position may then subsequently be used when the region of interest is imaged, since in the first table position the region of interest of the object is at a reference position inside the bore. This helps to provide for an efficient and intuitive workflow.

According to an embodiment of the invention, the imaging system is a magnetic resonance imaging system or a computed tomography system. The invention may be particularly advantageous for magnetic resonance imaging systems, since such systems often have long and narrow bores that are hard to reach into.

According to an embodiment of the invention, the controller is configured to acquire an off-center image of the object, acquired by the imaging system, when the table is in the third table position. The controller is configured to geometrically correct the off-center image. This may be particularly advantageous when the third table position is further outside a comparatively long magnetic resonance imaging system bore compared to the first table position, in order to improve access to the object, but close enough such that imaging of the region of interest is still possible. The region of interest of the object on the table is not at an isocenter of the bore when the table is in the third position but may in this way still be imaged. Furthermore, parts of the object outside the region of interest may also be imaged in the third position, which may be advantagous to locate e.g. a catheter, such as in cases when the catheter is not at the expected location. Access to the object may be improved in the third table position as compared to the first table position. It is therefore advantageous if the object can also be imaged when table is in the third table position. Off-center imaging may lead to distortions and signal voids of the region of interest. By receiving an off-center image of the object, i.e. an image wherein the imaged part of the object is not at an isocenter of the bore, and geometrically correct said image, it may be possible to image the procedure with geometrically correct images also when the table is in the ergonomically advantageous third position. Correction of geometric differences when the table is in the third table position may be fine-tuned based on differences of acquired images in the third table position as compared to images acquired in the first table position.

According to an embodiment of the invention, the imaging system is a magnetic resonance imaging system and the geometric correction of the off-center image comprises correcting for gradient non-linearities of the magnetic resonance system and/or comprises comparing the off-center image with an image acquired with the table at the first table position. Based on e.g. system characteristics, gradient non-linearity etc., it may be possible to calculate and set e.g. B0 shim, limiting image off-center values etc.

According to an embodiment of the invention, the imaging system comprises a display, and the controller is configured to provide the image on the display. This embodiment is advantageous since the system may directly present the image of the region of interest on the display, such that a user may provide input. The display may be positioned e.g. on the bore or in proximity to the bore of the imaging system such that it may be viewed when standing next to the imaging system. Alternatively, or additionally the system may comprise a display located remotely, e.g in another room or at another site.

According to an embodiment of the invention, the imaging system comprises a user interface. The user interface may include e.g. a speech interface, a gesture recognition interface, a touch interface, a mouse, a hand switch, or a foot switch. The user interface may advantageously be used to provide input to e.g. the table position, the intervention location, selection of imaging parameters etc. In case the system comprises a display, a user may interact with e.g. an image on the display via the user interface.

According to an embodiment of the invention, the imaging system comprises a camera directed towards the table, and the controller is configured to update a stored table position and/or a stored position of an object on the table based on an image from the camera. The camera may be e.g. a 2D or 3D camera, a camera registering visible and/or infrared radiation etc. The camera may advantageously improve the efficiency and accuracy of the workflow by determining the position of the object on the table with respect to the table, the table position e.g. with respect to the bore etc. As one example, the camera may provide input with respect to a change of position e.g. in case the object moves on the table. In this way, the accuracy of the workflow, such as the accuracy of the table positions, may be improved.

According to a second aspect of the invention, there is provided a computer implemented method for controlling a table with respect to an imaging system comprising a bore and a position indicator, the method comprising:
- receiving an image, acquired with the imaging system, of a region of interest of an object on the table, wherein the image is acquired with the table positioned in a stored first table position, wherein in the first table position the region of interest of the object is at a stored reference position inside the bore;
- receiving user input marking an intervention location in the image;
- determining a puncture location on the object relative to the reference position;
- determining a second table position based on the puncture location, a stored position indicated by the position indicator, and the first table position, such that when the table is in the second table position the puncture location on the object coincides with the position indicated with the position indicator; and
- storing a third table position, wherein when the table is in the third table position the table is further outside of the bore compared to in the first position, and wherein the region of interest of the object on the table in the third table position is not at an isocenter of the bore.

According to an embodiment of the invention, the method further comprises:
- storing a temporary table position, wherein in the temporary table position the region of interest on the object coincides with the position indicated with the position indicator, and
- determining the first table position based on the temporary table position and an offset between the position indicated with the position indicator and the reference position inside the bore.

According to an embodiment of the invention, the step of determining a puncture location on the object relative to the reference position comprises road-mapping of an internal volume of the object and/or determining the puncture location (from the intervention location) using a trained artificial intelligence algorithm. Determination of the puncture location, such as via a translation from an intervention location in the image to a puncture location, may comprise to calculate, based on an imaged volume of the object, an optimal path for an intervention needle, catheter or similar instrument from the puncture location on the object to the intervention location inside the object. Translating the intervention location in the image to a puncture location on the object may be based on anatomical regions that can be passed or needs to be avoided by e.g. a catheter. Determination of a puncture location may include the use of artificial intelligence algorithms, such that algorithms that can learn from previous intervention procedures and/or strategies. Computational road-mapping may e.g. automatically determine the puncture location or provide recommendations as to suitable options.

According to a third aspect of the invention, there is provided a computer program element, which, when being executed by a controller, is adapted to cause the controller to perform the method as described above. The computer program element may be software made available for downloading from a server, e.g. via the internet.

According to a fourth aspect of the invention, there is provided a computer-readable medium having stored thereon the computer program element.

These and other aspects of the invention will be apparent from and elucidated with reference to the embodiments described hereinafter.

### BRIEF DESCRIPTION OF THE DRAWINGS

Fig. 1 schematically illustrates an imaging system.
Fig. 2 schematically illustrates a workflow with an imaging system.
Fig. 3 schematically illustrates a workflow with an imaging system.
Fig. 4 schematically illustrates needle positioning with respect to a lesion.
Fig. 5 shows a flow diagram of a computer implemented method.
Fig. 6 shows a flow diagram of a computer implemented method.

### DETAILED DESCRIPTION OF EMBODIMENTS

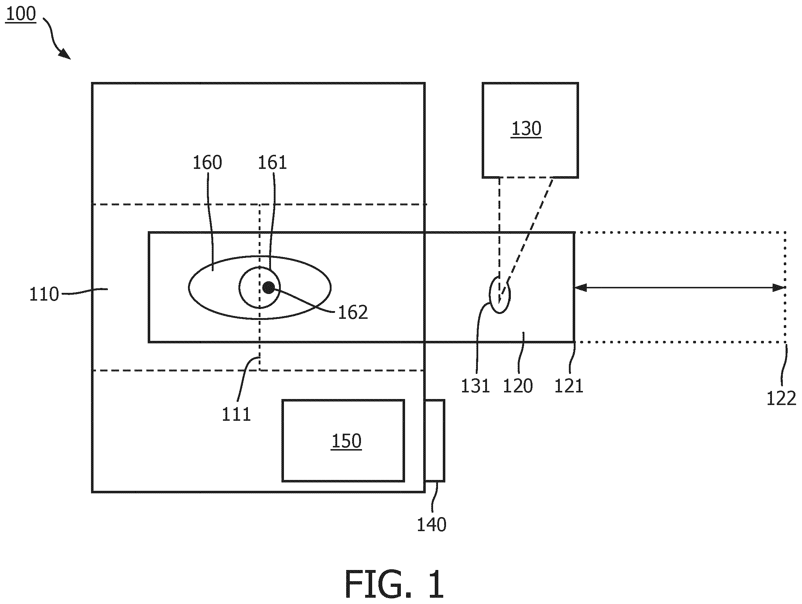

An imaging system 100, according to embodiments of the invention, is schematically illustrated in Fig. 1. The imaging system 100 includes a bore 110, which may be relatively long and narrow such that it can fit a patient or the parts of the patient to be imaged. An example of a type of imaging systems where a long and narrow bore so commonly used in order to achieve good imaging quality is magnetic resonance imaging systems. However, also other tomographic imaging systems, such as computed tomography systems, positron emission tomography systems, single-photon emission computed tomography systems, combinations of such systems etc. may also have long and narrow bores. In the bore 110 of the imaging system 100, a reference position 111 is defined. The reference position may be e.g. at an isocenter of the bore or close to the center of the bore.

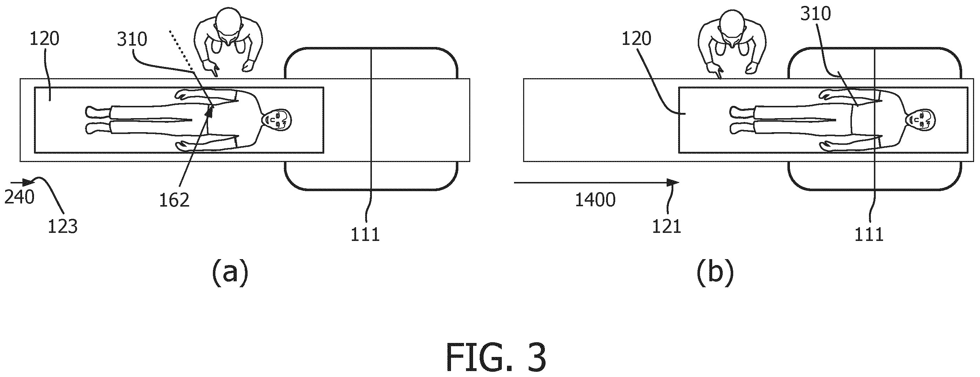

The imaging system 100 includes a table 120. The table may move longitudinally in and out of the bore or closer to or further away from the bore. An object 160 to be imaged, such as a patient to be imaged, may be positioned on the table 120. The table is configured to move between at least a first table position 121, a second table position 122 and a third table position 123 (not shown in Fig. 1 but in Fig. 3). The table positions are defined, e.g. with one or more coordinates in relation to the reference position 111 in the bore.

The imaging system includes a position indicator 130 that is configured to indicate a position 131. The indicated position 131, such as the center point or center line of the indicated position 131, may also be defined in the same coordinate system as the table positions and the reference position 111. The indicated position may e.g. take the form of a line or a dot or a circle or an oval etc. In the schematic drawing in Fig. 1, the indicated position 131 is visible on the table 120. However, if an object 160 on the table is located at the position 131, the position indicator will mark a location 131 on the object. The indicated position 131 may be fixed in the coordinate system, such as if the position is indicated with a laser bow that is fixed with respect to the imaging system. Alternatively, the indicated position 131 may be altered by the position indicator 130, such that positions at various coordinates may be indicated. The coordinate system for the positions with respect to imaging system 100 may be a one-dimensional coordinate system along the longitudinal axis of the table 120. Other coordinate systems are also possible, such as e.g. a two-dimensional system in a plane of the table 120.

The imaging system includes a controller 150. The controller may e.g. include an ASIC, a FPGA, and/or a processor and a memory storing instructions for controlling the processor. In the example in Fig. 1, the imaging system includes a local display 140. The imaging system may in some examples also include a user interface (not shown in Fig. 1). The user interface may include e.g. a speech interface, a gesture recognition interface, a touch interface, a mouse, a hand switch, or a foot switch. Such a user interface may be used to provide user input to e.g. the table position, the intervention location, selection of imaging parameters etc. In case the system comprises a display, like in Fig. 1, and a user interface, a user may interact with e.g. an image on the display via the user interface.

Fig. 1 illustrates an object 160, such as a patient, on table 120 in the bore 110. The object 160 has a region of interest 161 that may be imaged by the imaging system 100. Based on imaging of the region of interest 161 and user input marking an intervention location in at least one image, the controller 150 may determine a puncture location 162 in the coordinate system that is relative to the reference position 111. When the puncture location 162 is known, the second table position 122 may be determined such that when the table 120 is in the second table position 122, the puncture location 162 is at the position 131 indicated by the position indicator 130. Furthermore, the controller 150 is configured to store the third table position 123, which provides an improved working position as e.g. illustrated in Fig. 3.

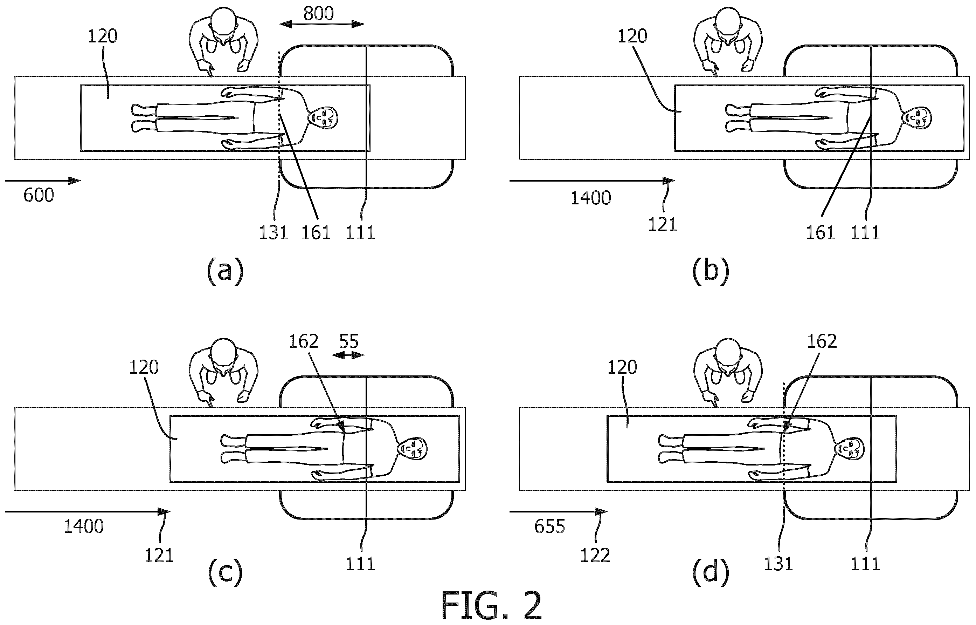

Fig. 2 shows an example of a workflow with the imaging system 100. The positions in Fig. 2 are illustrated with one-dimensional coordinates from the leftmost side of the imaging system 100 and from the reference position 111. In this case, the position indicator 130 is a stationary light visor with a fixed indicated position 131.

In Fig. 2, at (a), the table has been moved to a table position with coordinate 600, where the region of interest 161 of the patient is below the position 131 indicated by the light visor. Based on the table coordinate and the distance between the reference position 111 and the indicated position 131, shown as 800 in Fig. 2, at (a), a first table position 121 may be determined and stored. In this case the first table position 121 has coordinate 1400.

Consequently, as shown in Fig. 2, at (b), when the table with the patient has been moved to the first table position 121 (with coordinate 1400), the region of interest 161 of the patient is positioned at the reference position 111. The patient has moved further into the bore 110. In this example, the reference position is at the isocenter of the bore 110. With the region of interest 161 at the isocenter of the bore 110, the patient may be imaged by the imaging system 100 to generate one or multiple images of the region of interest.

A user, such as an interventionalist, may review and interact with the generated images in viewing software. Based on input from the user an entry point or puncture location 162 may be determined, as illustrated in Fig. 2 at (c). With the determined puncture location 162 in relation to the reference position 111, here shown as a difference of 55 distance units, and the known coordinate of the light visor in relation to the reference position (here -800), a second table position 122 may be determined (at 655) and stored.

Fig. 2, at (d) shows the table in the second table position 122. Now the puncture location 162 is directly underneath the position 131 indicated by the light visor, which may assist the interventionalist in quickly finding the right location and performing an intervention or part of an intervention, such as placing a needle at the puncture location 162. For simplicity, the location 131 is here indicated with a line. However, e.g. indication of a spot, such as in combination with a two-dimensional coordinate system is also possible.

Fig. 3 illustrates additional steps of a workflow, such as the workflow shown in Fig. 2. In Fig. 3, at (a), the table with the patient has been moved further out of the bore 110 to the third table position 123. The system may store the coordinate (here 240) of the third table position in the memory. This third table position 123 is a position which may be more comfortable and ergonomic for the interventionalist to perform an intervention without having to stretch into the bore 110 or be limited by a position underneath the light visor. In the schematic of Fig. 3, the interventionalist or other user is placing a needle 310 at the puncture location 162.

It is noted that in the example in Fig. 3, the third position 123 is shown as located far out from the bore to provide for a position with optimal freedom of movement for the interventionalist. However, the third table position 123 may be located closer to the first table position 121, such as closer to the iso-center compared to the second position 122. This may advantageously limit table movement and/or enable off-center imaging. Ergonomics is still improved for the interventionalist who does not have to stretch as far into the bore to access the patient as in the first table position 121.

In Fig. 3, at (b), the table has been moved back to the first table position 121. In this position the region of interest 161, here with a needle 310, may be imaged with the imaging system 100 in order to check or confirm progress of the intervention. If there is a display on or close by the imaging system 100, the interventionalist may be able to view the images without having to move to a different location. As both the first table position 121 and the third table position 123 are stored, the user of the imaging system 100 may easily toggle the table between a comfortable working position and an imaging position. The table may be automatically moved between the first 121 and third 123 table positions, such as at the command of the user via a user interface.

As an example, an operator of e.g. a magnetic resonance imaging (MRI) system with a light visor, may define with the system one or more of the following table positions:
1. The area of interest indicated by the light visor in the isocenter or close to the isocenter.
2. The entry point for a needle as indicated in an MRI scan, is underneath the laser (such as a standard laser of the scanner or a laser bridge in an MRI-radiation therapy setup)
3. The entry point of the needle outside the magnet at a comfortable working location for the interventional operator
4. The entry point of a catheter at a location on the patient that is not covered by the roadmap scan that depicts the lesion, is at the isocenter or close to the isocenter

These positions may be indicated on a touch screen at the magnet and the operator can use e.g. buttons, gesture control or voice control to instruct the table to travel to the desired location.

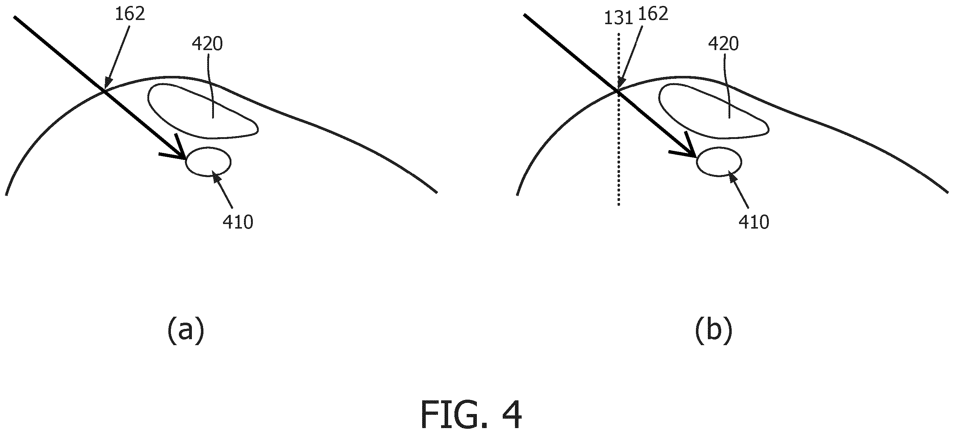

Fig. 4 schematically shows an example of a part of a patient anatomy with a needle entry position or puncture location 162, in relation to a lesion 410 to be treated. The interventional radiologist may want to puncture a lesion in an organ such as for instance the liver or kidney. The patient may be moved into the bore of the imaging system (table in first position 121) and a roadmap scan is made of the region of interest, where the roadmap scan shows the location of the lesion 410. Using one or multiple images from the scan, a plan can be made of what the best location is to enter the body with a needle, catheter or similar. The location of the lesion 410 is translated to a puncture location 162. To avoid sensitive areas 420, this may not be the straight, shortest trajectory but e.g. under a certain angle, and/or with a curved trajectory etc. Translation from the intervention location to a puncture location 162, taking anatomical information to pass or to avoid passing areas of the patient anatomy, may leverage artificial intelligence. In a second step, as illustrated in Fig. 4 (b), the table may be moved to a second table position 122, such that entry point 162 is being marked with the position location 131 on the skin.

As a further step, not shown in Fig. 4, the patient may be moved out of the bore a bit further (e.g. to third table position 123) to conveniently prepare the skin, apply local anesthesia, create a small incision and place the needle. Once the needle is inserted for a few mm, the patient may be moved inside the scanner again (first table position 121) and with imaging the correct placement and direction can be confirmed. The patient may be moved out again to the work position (third table position 123) to advance the needle in the right direction based on the information taken from the imaging scans. Such a process of toggling between imaging position and working position may be repeated until the needle has reached the target as confirmed by imaging system.

Several variations to the workflows with the imaging system are conceivable. E.g. as mentioned above, the table may in the third table position 123 be positioned in an ergonomic image guidance position that is at an image off center position where overview images of the region of interest 161 may be provided. The overview images at such an image guidance position may be geometrically corrected, e.g. by taking other overview images into account. In the case of magnetic resonance imaging, the geometric correction may take gradient magnet non-linearities into account.

A surgeon's commands to e.g. scan, reposition table, toggle the table between positions, etc. may be determined by the system using a bore microphone, camera, (foot) switch, touch screen or similar. The surgeon's commands may be used to guide catheter placement, e.g. via catheter guidance robotics using such user interfaces.

The catheter position may be tracked and visualized on an in/on bore screen. The surgeon may initiate scanning, table repositioning between the initial catheter placing position, an ergonomic image off-center position or an isocenter position by e.g. vocal commands or gestures. Such as with artificial intelligence-based speech and/or gesture recognition.

Any part of the workflow may include the use of catheter guidance robotics, e.g. instead of manual catheter placement.

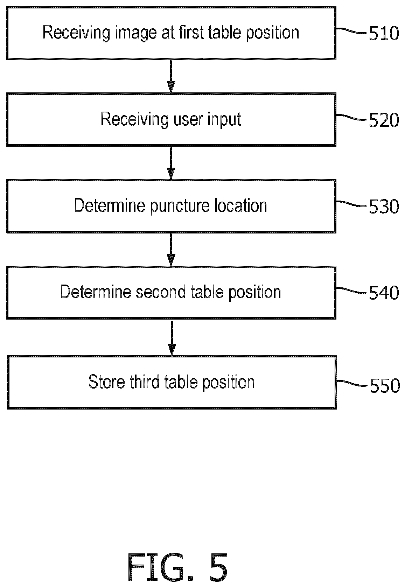

Fig. 5 illustrates an example of a computer-implemented method with a flow chart. The computer-implemented method is suitable for controlling a table 120 with respect to an imaging system 100 comprising a bore 110 and a position indicator 130. The method includes:
- receiving 510 an image, acquired with the imaging system 100, of a region of interest 161 of an object 160 on the table 120, wherein the image is acquired with the table 120 positioned in a stored first table position 121, wherein in the first table position 121 the region of interest 161 of the object 160 is at a stored reference position 111 inside the bore 110;
- receiving 520 user input marking an intervention location in the image;
- determining 530 a puncture location 162 on the object relative to the reference position 111;
- determining 540 a second table position 122 based on the puncture location 162, a stored position 131 indicated by the position indicator 130, and the first table position 121, such that when the table 120 is in the second table position 122 the puncture location 162 on the object coincides with the position indicated 131 with the position indicator 130; and
- storing 550 a third table position 123, wherein when the table is in the third table position 123 the table is further outside of the bore 110 compared to in the first position 121, and wherein the region of interest 161 of the object on the table in the third table position 123 is not at an isocenter of the bore.

The third table position 123 may be predetermined and may be stored 550 prior to one or more of the other steps of the flow-chart in Fig. 5. The third table position 123 may be received or calculated or adapted when carrying out the method, e.g. from selection or other input by a user. The third table position 123 may be selected or calculated based on ergonomic parameters, such as a length of the user, a reach of the user, a height of the table, a length of the object, a length of an instrument etc.

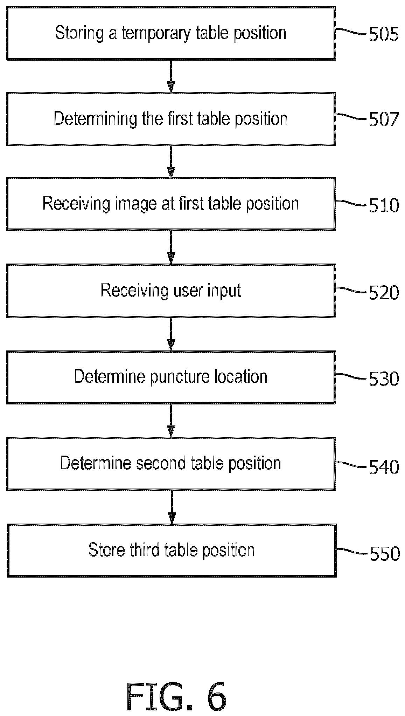

Fig. 6 shows an example of a method that is similar to the method in Fig. 5. However, in this example the method includes two additional steps. The method includes:
- storing 505 a temporary table position, wherein in the temporary table position the region of interest 161 on the object 160 coincides with the position indicated 131 with the position indicator 130;
- determining 507 the first table position 121 based on the temporary table position and an offset between the position indicated 131 with the position indicator 130 and the reference position 111 inside the bore 110;
- receiving 510 an image, acquired with the imaging system 100, of a region of interest 161 of an object 160 on the table 120, wherein the image is acquired with the table 120 positioned in a stored first table position 121, wherein in the first table position 121 the region of interest 161 of the object 160 is at a stored reference position 111 inside the bore 110;
- receiving 520 user input marking an intervention location in the image;
- determining 530 a puncture location 162 on the object relative to the reference position 111;
- determining 540 a second table position 122 based on the puncture location 162, a stored position 131 indicated by the position indicator 130, and the first table position 121, such that when the table 120 is in the second table position 122, the puncture location 162 on the object coincides with the position indicated 131 with the position indicator 130; and
- storing 550 a third table position 123, wherein when the table is in the third table position 123 the table is further outside of the bore 110 compared to in the first position 121, and wherein the region of interest 161 of the object on the table in the third table position 123 is not at an isocenter of the bore.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. The word "comprising" does not exclude the presence of elements or steps other than those listed in a claim. The word "a" or "an" preceding an element does not exclude the presence of a plurality of such elements. The invention may be implemented by means of hardware comprising several distinct elements, and/or by means of a suitably programmed processor. In the device claim enumerating several means, several of these means may be embodied by one and the same item of hardware. Measures recited in mutually different dependent claims may advantageously be used in combination.

## Claims

1. Imaging system (100) for medical imaging, the imaging system comprising:
a bore (110) for receiving an object (160) to be imaged;
a table (120) for supporting the object (160) and configured to move longitudinally with respect to the bore (110);
a position indicator (130) for indicating a position (131); and
a controller (150) configured to:
- receive (510) an image, acquired with the imaging system (100), of a region of interest (161) of the object (160) on the table (120), wherein the image is acquired with the table positioned in a stored first table position (121), wherein in the first table position (121) the region of interest (161) of the object (160) is at a reference position (111) inside the bore;
- receive (520) user input marking an intervention location in the image;
- determine (530) a puncture location (162) on the object relative to the reference position (111);
- calculate and store (540) a second table position (122), based on the puncture location (162), the position (131) indicated by the position indicator, and the stored first table position (121), such that when the table is in the second table position (122), the puncture location (162) on the object coincides with the position (131) indicated with the position indicator (130); and
**characterized in that** the controller (150) is further configured to:
- store a third table position (123), wherein when the table is in the third table position (123) the table is further outside of the bore (110) compared to in the first position (121), and wherein the region of interest (161) of the object on the table in the third table position (123) is not at an isocenter of the bore.

2. The imaging system according to claim 1, wherein the controller (150) is configured to calculate and store an updated coordinate for the position indicator (130), such that when the table is in the second table position (122) and the position indicator (130) indicates an updated position (131) with the updated coordinate, the puncture location (162) on the object coincides with the updated position (131) indicated with the position indicator (130).

3. The imaging system according to claim 1 or 2, wherein the controller (150) is configured to store (505) a temporary table position, wherein when the table is in the temporary table position the region of interest (161) coincides with the position (131) indicated with the position indicator (130), and
wherein the controller (150) is configured to calculate and store (507) the first table position (121) based on the temporary table position and an offset between the position (131) indicated with the position indicator (130) and the reference position (111).

4. The imaging system according to any of the preceding claims, wherein the imaging system (100) is a magnetic resonance imaging system or a computed tomography system.

5. The imaging system according to any of the preceding claims, wherein the controller (150) is configured to receive an off-center image of the object (160), acquired by the imaging system when the table (120) is in the third table position (123), and wherein the controller (150) is configured to geometrically correct the off-center image.

6. The imaging system according to claim 5, wherein the imaging system (100) is a magnetic resonance imaging system, and wherein the geometric correction of the off-center image comprises correcting for gradient non-linearities of the magnetic resonance imaging system and/or the geometric correction comprises comparing the off-center image with an image acquired with the table at the first table position (121).

7. The imaging system according to any of the preceding claims, wherein the imaging system comprises a display (140), and wherein the controller is configured to provide the image on the display (140).

8. The imaging system according to any of the preceding claims, wherein the imaging system comprises a user interface.

9. The imaging system according to any of the preceding claims, wherein the imaging system comprises a camera directed towards the table (120), and wherein the controller is (150) configured to update a stored table position and/or a stored position of an object (160) on the table based on an image from the camera.

10. A computer-implemented method for controlling a table (120) with respect to an imaging system (100) comprising a bore (110) and a position indicator (130), the method comprising:
- receiving (510) an image, acquired with the imaging system, of a region of interest of an object on the table, wherein the image is acquired with the table positioned in a stored first table position (121), wherein in the first table position (121) the region of interest (161) of the object is at a reference position (111) inside the bore;
- receiving (520) user input marking an intervention location in the image;
- determining (530) a puncture location (162) on the object relative to the reference position (111);
- determining (540) a second table position (121) based on the puncture location (162), a stored position (131) indicated by the position indicator (130), and the first table position (121), such that when the table is in the second table position (122), the puncture location on the object coincides with the position (131) indicated with the position indicator (130); and **characterized by**
- storing (550) a third table position (123), wherein when the table is in the third table position (123) the table is further outside of the bore (110) compared to in the first position (121), and wherein the region of interest (161) of the object on the table in the third table position (123) is not at an isocenter of the bore.

11. The method of claim 10, wherein the method further comprises:
- storing (505) a temporary table position, wherein in the temporary table position the region of interest on the object coincides with the position indicated with the position indicator, and
- determining (507) the first table position based on the temporary table position and an offset between the position indicated with the position indicator and the reference position inside the bore.

12. The method of claim 10 or 11, wherein determining (530) the puncture location comprises road-mapping of an internal volume of the object and/or determining the puncture location using a trained artificial intelligence algorithm.

13. A computer program element, which, when being executed by a controller, is adapted to cause the controller to perform the method according to claim 10 or 11 or 12.

14. A computer-readable medium having stored thereon the computer program element of claim 13.

## Patentansprüche

1. Bildgebungssystem (100) für medizinische Bildgebung, wobei das Bildgebungssystem umfasst:
eine Bohrung (110) zum Aufnehmen eines abzubildenden Objekts (160);
einen Tisch (120) zum Tragen des Objekts (160), und konfiguriert, um sich in Bezug auf die Bohrung (110) in Längsrichtung zu bewegen;
einen Positionsindikator (130) zum Angeben einer Position (131); und
eine Steuereinheit (150), die konfiguriert ist, um:
- ein mit dem Bildgebungssystem (100) erfasstes Bild eines Bereichs von Interesse (161) des Objekts (160) am Tisch (120) zu empfangen (510), wobei das Bild mit dem in einer gespeicherten ersten Tischposition (121) positionierten Tisch erfasst wird, wobei sich in der ersten Tischposition (121) der Bereich von Interesse (161) des Objekts (160) in einer Referenzposition (111) innerhalb der Bohrung befindet;
- eine Benutzereingabe zu empfangen (520), die eine Eingriffsstelle in dem Bild markiert;
- eine Einstichstelle (162) an dem Objekt in Bezug zur Referenzposition (111) zu bestimmen (530);
- eine zweite Tischposition (122), basierend auf der Einstichstelle (162), der durch den Positionsindikator angegebenen Position (131 ) und der gespeicherten ersten Tischposition (121) zu berechnen und zu speichern (540), sodass, wenn sich der Tisch in der zweiten Tischposition (122) befindet, die Einstichstelle (162) am Objekt mit der mit dem Positionsindikator (130) angegebenen Position (131) übereinstimmt; und
**dadurch gekennzeichnet, dass** die Steuereinheit (150) weiter konfiguriert ist, um:
- eine dritte Tischposition (123) zu speichern, wobei, wenn sich der Tisch in der dritten Tischposition (123) befindet, sich der Tisch im Vergleich zur ersten Position (121) weiter außerhalb der Bohrung (110) befindet, und wobei sich der Bereich von Interesse (161) des Objekts am Tisch in der dritten Tischposition (123) nicht in einem Isozentrum der Bohrung befindet.

2. Bildgebungssystem nach Anspruch 1, wobei die Steuereinheit (150) konfiguriert ist, um eine aktualisierte Koordinate für den Positionsindikator (130) zu berechnen und zu speichern, sodass, wenn sich der Tisch in der zweiten Tischposition (122) befindet und der Positionsindikator (130) eine aktualisierte Position (131) mit der aktualisierten Koordinate angibt, die Einstichstelle (162) am Objekt mit der mit dem Positionsindikator (130) angegebenen aktualisierten Position (131) übereinstimmt.

3. Bildgebungssystem nach Anspruch 1 oder 2, wobei die Steuereinheit (150) konfiguriert ist, um eine vorübergehende Tischposition zu speichern (505), wobei, wenn sich der Tisch in der vorübergehenden Tischposition befindet, der Bereich von Interesse (161) mit der mit dem Positionsindikator (130) angegebenen Position (131) übereinstimmt, und
wobei die Steuereinheit (150) konfiguriert ist, um die erste Tischposition (121) basierend auf der vorübergehenden Tischposition und einem Versatz zwischen der mit dem Positionsindikator (130) angegebenen Position (131) und der Referenzposition (111) zu berechnen und zu speichern (507).

4. Bildgebungssystem nach einem der vorstehenden Ansprüche, wobei das Bildgebungssystem (100) ein Magnetresonanz-Bildgebungssystem oder ein Computertomographie-System ist.

5. Bildgebungssystem nach einem der vorstehenden Ansprüche, wobei die Steuereinheit (150) konfiguriert ist, um ein außer-mittiges Bild des Objekts (160) zu empfangen, das von dem Bildgebungssystem erfasst wird, wenn sich der Tisch (120) in der dritten Tischposition (123) befindet, und wobei die Steuereinheit (150) konfiguriert ist, um das außer-mittige Bild geometrisch zu korrigieren.

6. Bildgebungssystem nach Anspruch 5, wobei das Bildgebungssystem (100) ein Magnetresonanz-Bildgebungssystem ist, und wobei die geometrische Korrektur des außer-mittigen Bildes Korrigieren von Gradienten-Nichtlinearitäten des Magnetresonanz-Bildgebungssystems umfasst und/oder die geometrische Korrektur Vergleichen des außer-mittigen Bildes mit einem Bild umfasst, das mit dem Tisch an der ersten Tischposition (121) erfasst worden ist.

7. Bildgebungssystem nach einem der vorstehenden Ansprüche, wobei das Bildgebungssystem eine Anzeige (140) umfasst, und wobei die Steuereinheit konfiguriert ist, um das Bild auf der Anzeige (140) bereitzustellen.

8. Bildgebungssystem nach einem der vorstehenden Ansprüche, wobei das Bildgebungssystem eine Benutzeroberfläche umfasst.

9. Bildgebungssystem nach einem der vorstehenden Ansprüche, wobei das Bildgebungssystem eine auf den Tisch (120) gerichtete Kamera umfasst, und wobei die Steuereinheit (150) konfiguriert ist, um eine gespeicherte Tischposition und/oder eine gespeicherte Position eines Objekts (160) am Tisch basierend auf einem Bild von der Kamera zu aktualisieren.

10. Computerimplementiertes Verfahren zum Steuern eines Tisches (120) in Bezug auf ein Bildgebungssystem (100), das eine Bohrung (110) und einen Positionsindikator (130) umfasst, wobei das Verfahren umfasst:
- Empfangen (510) eines mit dem Bildgebungssystem erfassten Bildes eines Bereichs von Interesse eines Objekts am Tisch, wobei das Bild mit dem in einer gespeicherten ersten Tischposition (121) positionierten Tisch erfasst wird, wobei sich in der ersten Tischposition (121) der Bereich von Interesse (161) des Objekts in einer Referenzposition (111) innerhalb der Bohrung befindet;
- Empfangen (520) einer Benutzereingabe, die eine Eingriffsstelle in dem Bild markiert;
- Bestimmen (530) einer Einstichstelle (162) an dem Objekt in Bezug zur Referenzposition (111);
- Bestimmen (540) einer zweiten Tischposition (121) basierend auf der Einstichstelle (162), einer durch den Positionsindikator (130) angegebenen gespeicherten Position (131) und der ersten Tischposition (121), sodass, wenn sich der Tisch in der zweiten Tischposition (122) befindet, die Einstichstelle am Objekt mit der mit dem Positionsindikator (130) angegebenen Position (131) übereinstimmt; und **gekennzeichnet durch**
- Speichern (550) einer dritten Tischposition (123), wobei, wenn sich der Tisch in der dritten Tischposition (123) befindet, sich der Tisch im Vergleich zur ersten Position (121) weiter außerhalb der Bohrung (110) befindet, und wobei sich der Bereich von Interesse (161) des Objekts am Tisch in der dritten Tischposition (123) nicht in einem Isozentrum der Bohrung befindet.

11. Verfahren nach Anspruch 10, wobei das Verfahren weiter umfasst:
- Speichern (505) einer vorübergehenden Tischposition, wobei in der vorübergehenden Tischposition der Bereich von Interesse am Objekt mit der mit dem Positionsindikator angegebenen Position übereinstimmt, und
- Bestimmen (507) der ersten Tischposition basierend auf der vorübergehenden Tischposition und einem Versatz zwischen der mit dem Positionsindikator angegebenen Position und der Referenzposition innerhalb der Bohrung.

12. Verfahren nach Anspruch 10 oder 11, wobei Bestimmen (530) der Einstichstelle Kartieren eines Innenvolumens des Objekts und/oder Bestimmen der Einstichstelle unter Verwendung eines trainierten Algorithmus künstlicher Intelligenz umfasst.

13. Computerprogrammelement, das, wenn es von einer Steuereinheit ausgeführt wird, angepasst ist, um die Steuereinheit zu veranlassen, das Verfahren nach Anspruch 10 oder 11 oder 12 durchzuführen.

14. Computerlesbares Medium, welches das darin gespeicherte Computerprogrammelement nach Anspruch 13 aufweist.

## Revendications

1. Système d'imagerie (100) pour l'imagerie médicale, le système d'imagerie comprenant :
un alésage (110) pour recevoir un objet (160) à imager ;
une table (120) pour supporter l'objet (160) et configurée pour se déplacer longitudinalement par rapport à l'alésage (110) ;
un indicateur de position (130) pour indiquer une position (131) ; et
un dispositif de commande (150) configuré pour :
- recevoir (510) une image, acquise avec le système d'imagerie (100), d'une région d'intérêt (161) de l'objet (160) sur la table (120), dans lequel l'image est acquise avec la table positionnée dans une première position de table stockée (121), dans lequel, dans la première position de table (121), la région d'intérêt (161) de l'objet (160) est à une position de référence (111) à l'intérieur de l'alésage ;
- recevoir (520) une entrée utilisateur marquant un emplacement d'intervention dans l'image ;
- déterminer (530) un emplacement de ponction (162) sur l'objet par rapport à la position de référence (111) ;
- calculer et stocker (540) une deuxième position de table (122), en fonction de l'emplacement de ponction (162), de la position (131) indiquée par l'indicateur de position et de la première position de table stockée (121), de telle sorte que lorsque la table est dans la deuxième position de table (122), l'emplacement de ponction (162) sur l'objet coïncide avec la position (131) indiquée par l'indicateur de position (130) ; et
**caractérisé en ce que** le dispositif de commande (150) est en outre configuré pour :
- stocker une troisième position de table (123), dans lequel lorsque la table est dans la troisième position de table (123), la table est plus à l'extérieur de l'alésage (110) par rapport à la première position (121), et dans lequel la région d'intérêt (161) de l'objet sur la table dans la troisième position de table (123) n'est pas à un isocentre de l'alésage.

2. Système d'imagerie selon la revendication 1, dans lequel le dispositif de commande (150) est configuré pour calculer et stocker une coordonnée mise à jour pour l'indicateur de position (130), de telle sorte que lorsque la table est dans la deuxième position de table (122) et que l'indicateur de position (130) indique une position mise à jour (131) avec la coordonnée mise à jour, l'emplacement de ponction (162) sur l'objet coïncide avec la position mise à jour (131) indiquée par l'indicateur de position (130).

3. Système d'imagerie selon la revendication 1 ou 2, dans lequel le dispositif de commande (150) est configuré pour stocker (505) une position de table temporaire, dans lequel, lorsque la table est dans la position de table temporaire, la région d'intérêt (161) coïncide avec la position (131) indiquée par l'indicateur de position (130), et
dans lequel le dispositif de commande (150) est configuré pour calculer et stocker (507) la première position de table (121) sur la base de la position de table temporaire et d'un décalage entre la position (131) indiquée par l'indicateur de position (130) et la position de référence (111).

4. Système d'imagerie selon l'une quelconque des revendications précédentes, dans lequel le système d'imagerie (100) est un système d'imagerie par résonance magnétique ou un système de tomodensitométrie.

5. Système d'imagerie selon l'une quelconque des revendications précédentes, dans lequel le dispositif de commande (150) est configuré pour recevoir une image décentrée de l'objet (160), acquise par le système d'imagerie lorsque la table (120) est dans la troisième position de table (123), et dans lequel le dispositif de commande (150) est configuré pour corriger géométriquement l'image décentrée.

6. Système d'imagerie selon la revendication 5, dans lequel le système d'imagerie (100) est un système d'imagerie par résonance magnétique, et dans lequel la correction géométrique de l'image décentrée comprend la correction des non-linéarités de gradient du système d'imagerie par résonance magnétique et/ou la correction géométrique comprend la comparaison de l'image décentrée avec une image acquise avec la table à la première position de table (121).

7. Système d'imagerie selon l'une quelconque des revendications précédentes, dans lequel le système d'imagerie comprend un écran (140), et dans lequel le dispositif de commande est configuré pour fournir l'image sur l'écran (140).

8. Système d'imagerie selon l'une quelconque des revendications précédentes, dans lequel le système d'imagerie comprend une interface utilisateur.

9. Système d'imagerie selon l'une quelconque des revendications précédentes, dans lequel le système d'imagerie comprend une caméra dirigée vers la table (120), et dans lequel le dispositif de commande (150) est configuré pour mettre à jour une position de table stockée et/ou une position stockée d'un objet (160) sur la table en fonction d'une image provenant de la caméra.

10. Procédé mis en œuvre par ordinateur pour commander une table (120) par rapport à un système d'imagerie (100) comprenant un alésage (110) et un indicateur de position (130), le procédé comprenant :
- la réception (510) d'une image, acquise avec le système d'imagerie, d'une région d'intérêt d'un objet sur la table, dans lequel l'image est acquise avec la table positionnée dans une première position de table stockée (121), dans lequel, dans la première position de table (121), la région d'intérêt (161) de l'objet est à une position de référence (111) à l'intérieur de l'alésage ;
- la réception (520) d'une entrée utilisateur marquant un emplacement d'intervention dans l'image ;
- la détermination (530) d'un emplacement de ponction (162) sur l'objet par rapport à la position de référence (111) ;
- la détermination (540) d'une deuxième position de table (121) en fonction de l'emplacement de ponction (162), d'une position stockée (131) indiquée par l'indicateur de position (130) et de la première position de table (121), de sorte que lorsque la table est dans la deuxième position de table (122), l'emplacement de ponction sur l'objet coïncide avec la position (131) indiquée par l'indicateur de position (130) ; et **caractérisé par**
- le stockage (550) d'une troisième position de table (123), dans lequel lorsque la table est dans la troisième position de table (123), la table est plus à l'extérieur de l'alésage (110) par rapport à la première position (121), et dans lequel la région d'intérêt (161) de l'objet sur la table dans la troisième position de table (123) n'est pas à un isocentre de l'alésage.

11. Procédé selon la revendication 10 dans lequel le procédé comprend en outre :
- le stockage (505) d'une position de table temporaire, dans lequel, dans la position de table temporaire, la région d'intérêt sur l'objet coïncide avec la position indiquée par l'indicateur de position, et
- la détermination (507) de la première position de table en fonction de la position de table temporaire et d'un décalage entre la position indiquée par l'indicateur de position et la position de référence à l'intérieur de l'alésage.

12. Procédé selon la revendication 10 ou 11, dans lequel la détermination (530) de l'emplacement de ponction comprend la cartographie d'un volume interne de l'objet et/ou la détermination de l'emplacement de ponction à l'aide d'un algorithme d'intelligence artificielle formé.

13. Élément programme informatique, qui, lorsqu'il est exécuté par un dispositif de commande, est conçu pour amener le dispositif de commande à exécuter le procédé selon la revendication 10 ou 11 ou 12.

14. Support lisible par ordinateur sur lequel est stocké l'élément programme informatique selon la revendication 13.
